# EUROPÄISCHE PATENTSCHRIFT

(11) **EP 0 831 151 B1**
(45) Veröffentlichungstag und Bekanntmachung des Hinweises auf die Patenterteilung: **18.12.2002**
(21) Anmeldenummer: 97250278.5
(22) Anmeldetag: 17.09.1997
(51) Int. Cl.: C12Q 1/00, G01N 33/48, C02F 3/12

(54) **Verfahren und Vorrichtung zur Messung der Nitrifikationsleistung von Belebtschlamm**
Process and apparatus for measuring the nitrification-efficiency of activated sludge
Procédé et dispositif de mesure du rendement de la nitrification des boues activées

(30) Priorität: 19.09.1996 DE 19640333
(43) Veröffentlichungstag der Anmeldung: 25.03.1998
(73) Patentinhaber: LAR Analytik und Umweltmesstechnik GmbH, D-10963 Berlin (DE)
(72) Erfinder: Pilz, Ulrich, 13465 Berlin (DE)
(74) Vertreter: Heinze, Ekkehard, Dipl.-Phys. Dr.

(56) Entgegenhaltungen:
- DE-A- 2 951 707
- DE-A- 3 932 640
- DATABASE WPI Section Ch, Week 9504 Derwent Publications Ltd., London, GB; Class D15, AN 95-027389 XP002056958 & JP 06 312 197 A (MEIDENSHA CORP)
- DATABASE WPI Section Ch, Week 9335 Derwent Publications Ltd., London, GB; Class D15, AN 93-277660 XP002056959 & JP 05 192 688 A (MEIDENSHA CORP)
- DATABASE WPI Section Ch, Week 9223 Derwent Publications Ltd., London, GB; Class D15, AN 92-188564 XP002056960 & JP 04 122 498 A (MEIDENSHA CORP)
- DATABASE WPI Section Ch, Week 9340 Derwent Publications Ltd., London, GB; Class D15, AN 93-316796 XP002056961 & JP 05 228 485 A (MEIDENSHA CORP)
- DATABASE WPI Section Ch, Week 9629 Derwent Publications Ltd., London, GB; Class D15, AN 96-281526 XP002056962 & JP 08 117 792 A (MEIDENSHA CORP)

## Beschreibung

Die Erfindung betrifft ein Verfahren zur Bestimmung der Nitrifikationsleistung von Belebtschlamm in einer wäßrigen Lösung (speziell aufzubereitendem Wasser bzw. Abwasser) und eine Vorrichtung zur Durchführung des Verfahrens.

Die Analyse von Wasser bzw. Abwasser hinsichtlich seiner Inhaltsstoffe und der bei der Aufbereitung/Entsorgung eingesetzten Hilfsmittel hinsichtlich ihrer spezifischen Leistungsfähigkeit sind unabdingbare Voraussetzungen für eine den hohen aktuellen Qualitätsanforderungen genügende und effiziente Wasseraufbereitung und Abwasserentsorgung.

Bei der Klärung von Abwässern aus dem privaten und gewerblichen Bereich stellt der (biologische) Abbau organischer Kohlenstoffverbindungen ein vordringliches Ziel dar. Im Zusammenhang mit der biologischen Aufbereitung ist der sich im biologischen Sauerstoffbedarf ausdrückende Gehalt an biologisch abbaubaren Stoffen eine ebenso entscheidende Größe wie der Metabolismus der für die Aufbereitung wesentlichen aeroben Bakterien.

Aus DE 29 51 707 C2 ist eine Einrichtung zur Bestimmung der Konzentration von biologisch abbaubaren Stoffen in Abwässern bekannt, mit der der biologische Sauerstoffbedarf des Abwassers (als Maß für diese Konzentration) über eine Vergleichsmessung der Sauerstoffkonzentrationsdifferenz am Ein- und Ausgang zweier parallel betriebener Reaktoren ermittelt wird.

Im Ergebnis des Abbaus der Kohlenstoffverbindungen, insbesondere als Folgeprodukt der bakteriellen Verwertung von Eiweißstoffen, bilden sich Stickstoffverbindungen (Ammonium), die in nachteiliger Weise eine Eutrophierung der Gewässer bewirken und eine Gefahr für den Fischbesatz darstellen. Diese Folgeprodukte sind daher im Rahmen der Abwasserreinigung ebenfalls zu entfernen. Die Entfernung des Ammoniums läuft in zwei Stufen ab: Zunächst wird Ammonium unter Veratmung von im Wasser gelöstem Sauerstoff ("aerob") durch spezielle Mikroorganismen ("Nitrifikanten") zu Nitrit und Nitrat oxidiert, und anschließend erfolgt unter Sauerstoffmangelbedingungen (quasi "anaerob") durch andere Mikroorganismen eine Reduktion des Nitrats ("Denitrifikation") zu gasförmigem Stickstoff, der ohne weiteres in die Atmosphäre entweichen kann.

Die an dem Nitrifikationsprozeß beteiligten Bakterien (Nitrifikanten) - der Gattungen Nitrobacter und Nitrosomonas - stellen diffizile Ansprüche an Nährstoffe, Temperatur, pH-Wert und weitere Umgebungsbedingungen und sind insbesondere sehr schadstoffempfindlich. Ihre Leistungsfähigkeit, die die Nitrifikationsfähigkeit eines Belebtschlamms bestimmt, muß daher möglichst kontinuierlich überwacht werden, um den Aufbereitungsprozeß in Abhängigkeit von dieser wichtigen Größe zeitnah steuern und insbesondere die Einflußfaktoren für die Nitrifikationsleistung selbst optimieren zu können. Hieraus ergibt sich der Bedarf nach einem Verfahren und einer Vorrichtung zu dessen Durchführung, mit denen der Metabolismus der besagten Bakterien und die hierdurch bestimmte Nitrifikationsleistung eines Belebtschlamms meßtechnisch kontinuierlich erfaßt werden kann.

Der Erfindung liegt die Aufgabe zugrunde, ein solches Verfahren und eine entsprechende Vorrichtung anzugeben.

Die Aufgabe wird hinsichtlich des Verfahrens durch ein Verfahren mit den Merkmalen des Anspruchs 1 und hinsichtlich der Vorrichtung durch eine Vorrichtung mit den Merkmalen des Anspruchs 11 gelöst.

Die Erfindung schließt den Gedanken ein, daß zur Bestimmung der Nitrifikationsleistung die Bestimmung des mit diesem Prozeß verbundenen Sauerstoffumsatzes, d.h. einer Sauerstoff-Massenbilanz, geeignet ist. Sie schließt weiter den Gedanken ein, daß zur Bestimmung dieses Sauerstoffumsatzes eine vergleichende Sauerstoffgehaltsmessung am Ein- und Augang eines den zu prüfenden Belebtschlamm enthaltenden biologischen Reaktors genutzt werden kann, wenn eine Korrektur des Meßergebnisses - das die bakterielle Gesamt-Respiration widerspiegelt - um verschiedene Anteile gelingt, die nicht den Sauerstoffverbrauch infolge der Nitrifikation ausdrücken, sondern andere Vorgänge im Reaktor. Solche Vorgänge sind zum einen die sogenannte endogene Atmung der Bakterien, d.h. die Aufrechterhaltung von deren reinen Lebensfunktionen (ohne Abbau von Kohlenstoffverbindungen oder Ammonium) und zum anderen der oben erwähnte Abbau der Kohlenstoffverbindungen.

Letztlich münden diese Überlegungen in die den Kern der vorliegenden Erfindung bildende Lehre, den Nitrifikations-Sauerstoffumsatz aufgrund einer vergleichenden Bestimmng des Sauerstoffumsatzes in zwei Reaktoren zu ermitteln, von denen in dem einen eine Nitrifikation stattfindet, während sie in dem anderen unterbunden ist, was eine Separation der verschiedenen Respirationsanteile ermöglicht.

In einer vorteilhaften Weiterbildung des Erfindungsgedankens erfolgt eine noch weitergehende Separation zur zusätzlichen Bestimmung des sogenannten biochemischen Sauerstoffbedarfs (BSB; nach dem englischsprachigen Terminus "biochemical oxygen demand" auch als "BOD" abgekürzt), indem ein dritter Reaktor eingesetzt wird, der statt mit Abwasser bzw. verunreinigtem Wasser mit reinem Wasser beschickt wird und in dem der Belebtschlamm daher ausschließlich eine endogene Atmung ausführen kann. Der BSB wird aus der Differenz des Sauerstoffumsatzes in dem Reaktor, in dem eine Umsetzung der Kohlenstoffverbindungen im Abwasser stattfindet, aber die Nitrifikation gehemmt ist, und in dem letztgenannten Reaktor mit ausschließlich endogener Atmung ermittelt.

Eine weitere Möglichkeit zur zusätzlichen Bestimmung des BSB bietet sich, wenn der Sauerstoffumsatz infolge endogener Atmung als bekannt vorausgesetzt werden kann: Dann kann auf den dritten Reaktor verzichtet und der BSB aufgrund einer Korrekturrechnung aus dem Sauerstoffumsatz im zweiten Reaktor (mit gehemmter Nitrifikation) ermittelt werden.

Zusätzliche Aussagemöglichkeiten bezüglich der organischen Wasser- bzw. Abwasserinhaltsstoffe ermöglicht eine weitere Abwandlung des Verfahrens, bei der mindestens in einem des ersten und zweiten Reaktionsgefäßes zusätzlich der Kohlendioxidgehalt über der wäßrigen Lösung oder kontinuierlich im Abluftstrom gemessen wird. Hieraus kann - natürlich wieder aufgrund der bekannten Mengen bzw. Volumina der Einsatzstoffe - eine Kohlenstoffbilanz gewonnen werden.

In dem Fall, daß der Sauerstoffgehalt des den Reaktionsgefäßen zugeführten Gasgemisches - bei Luft ca. 20% - nicht oder nicht genau genug bekannt ist, wird auch am Eingang der Reaktionsgefäße der Sauerstoffgehalt gemessen und die Auswertung aufgrund der Differenz zwischen ein- und ausgangsseitigem Sauerstoffgehalt vorgenommen.

Einer der großen praktischen Vorzüge des erfindungsgemäßen Verfahrens besteht darin, daß es insbesondere kontinuierlich durchgeführt werden kann, wobei die pro Zeiteinheit den Reaktionsgefäßen zugeführten Mengen der Einsatzstoffe - streng genommen, mit Ausnahme des Nitrifikationsinhibitors, dessen Menge keine Rolle spielt, solange sie zu einer sicheren Inhibierung der Nitrifikationsreaktion ausreicht - vorteilhaft über Mittel zur Durchflußsteuerung (etwa Dosierpumpen) oder über eine Durchflußmessung bestimmt werden.

Die Berechnungen, die möglichst zeitnahe Ergebnisse liefern sollen, werden vereinfacht und damit zeitlich verkürzt, wenn in zweckmäßiger Weise das erste und zweite und - falls ein solches vorgesehen ist - auch das dritte Reaktionsgefäß verfahrenstechnisch identisch ausgebildet sind. Dazu gehört auch, daß möglichst gleiche Einsatz- bzw. Durchflußmengen der Einsatzstoffe eingestellt werden.

Ein bewährter Wirkstoff, der beim vorliegenden Verfahren als Nitrifikationsinhibitor eingesetzt werden kann, ist Allylthioharnstoff (ATH).

Handelt es sich bei dem aufzubereitenden Wasser bzw. Abwasser um ein an organischer Substanz bzw. Ammonium besonders armes, könnte der Nährstoffgehalt für die Nitrifikanten limitierend auf die Nitrifikationsleistung wirken, was die Aussage hinsichtlich der durch den Metabolismus des Belebtschlamms bedingten Nitrifikationsfähigkeit verfälschen würde. In diesem Fall kann die Dosierung zusätzlicher Nährstoffe - Quellen organischen Kohlenstoffs (Peptone, Zucker, organsiche Säuren etc.) oder von Stickstoff (Ammonium oder Harnstoff) - in die erste und zweite Reaktorstrecke erforderlich werden.

Da die Nitrifikation ein temperatursensibler Prozeß ist, kommt es zur Gewinnung genauer und reproduzierbarer Ergebnisse darauf an, daß das Meßverfahren bei einer im wesentlichen konstanten und für die Reaktionsgefäße gleichen Temperatur abläuft. Bevorzugt wird es im Bereich zwischen 20°C und 40°C, noch spezieller zwischen 30°C und 35°C, durchgeführt.

Bei der Vorrichtung zur Durchführung des Verfahrens sind in einer vorteilhaften Weiterbildung ein drittes Reaktionsgefäß, dem Zufuhreinrichtungen zum Zuführen von reinem Wasser, Belebtschlamm und Luft oder Sauerstoff zugeordnet sind, und ein dem dritten Reaktionsgefäß zugeordneter dritter Sauerstoffühler vorgesehen. Die Auswertungseinrichtung ist dann zusätzlich zur Ermittlung des biochemischen Sauerstoffbedarfs ausgebildet und umfaßt eine Subtraktionseinheit zur Differenzbildung aus den Ausgangssignalen des zweiten und dritten Sauerstoffühlers oder aus von diesen abgeleiteten Signalen. Wie auch bei der im Vorrichtungs-Hauptanspruch spezifizierten Vorrichtung wird die Auswertungseinrichtung vorzugsweise als Mikrorechner bzw. PC ausgeführt sein.

Die Reaktionsgefäße sind zweckmäßigerweise kontinuierlich durchströmte Systeme mit sogenannter "Plug-flow"-oder Kolbenströmungs-Charakteristik - etwa Schlauchreaktoren oder jeweils eine Reihe hintereinandergeschalteter Rührreaktoren - , bei denen eine nur geringe Längsvermischung des Flüssigkeits-/Luft-Gemischs stattfindet. Sie haben bevorzugt gleichen Aufbau und gleiche Abmessungen.

In der Ausführung der Vorrichtung, die auch eine Erstellung einer Kohlenstoffbilanz erlaubt, ist mindestens einem des ersten und zweiten Reaktionsgefäßes ein Kohlendioxidfühler zugeordnet und die Auswertungseinrichtung mit diesem verbunden und zur Bestimmung der Kohlenstoffbilanz ausgebildet. Auch dies wird in einer PC-Konfiguration softwaremäßig realisiert sein.

Beim Betrieb an einer Wasser- oder Abwasseraufbereitungsanlage ist die Vorrichtung in zweckmäßiger Weise auf der Einlaufseite des ersten und zweiten Reaktionsgefäßes über ein Probenahmeröhrchen direkt mit einer Wasser- oder Abwasserleitung verbunden.

Die Reaktionsgefäße sind - bevorzugt gemeinsam - in einem Thermostaten untergebracht, dessen Temperatur insbesondere in dem oben erwähnten T-Bereich steuerbar ist.

In zum automatischen Betrieb geeigneter Ausführung verfügt die Vorrichtung über eine Ablaufsteuereinheit, die insbesondere die Zufuhr der Einsatzstoffe und den Betrieb der Auswertungseinrichtung sowie wahlweise die Verfahrenstemperatur steuert.

Die Vorrichtung wird vorteilhaft bei einem Wasserversorgungs- oder Abwasserentsorgungssystem eingesetzt, um dieses aufgrund der gewonnenen Auswertungsergebnisse zu steuern. Dann umfaßt die Auswertungseinrichtung einen Vergleichswertspeicher und eine Vergleichereinheit zur Ausführung von Soll/Ist-Vergleichen oder Vergleichen aktueller Werte mit Vergangenheitswerten. Ihr Ausgang ist dann an die Steuereinheit der Prozeßsteuerung dieses Systems angebunden, die zur Ausgabe von Steuersignalen an Stellglieder, insbesondere Schieber- oder Dosiermittel-Stelleinrichtungen, in der Wasserversorgungs- oder Abwasserentsorgungsanlage ausgebildet ist.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Vorrichtung zur Durchführung des erfindungsgemäßen Verfahrens in einer ersten Ausführungsform,
- Figur 2: eine Vorrichtung zur Durchführung des erfindungsgemäßen Verfahrens in einer zweiten Ausführungsform und
- Figur 3: ein Funktions-Blockschaltbild als Detaildarstellung zu Fig. 1.

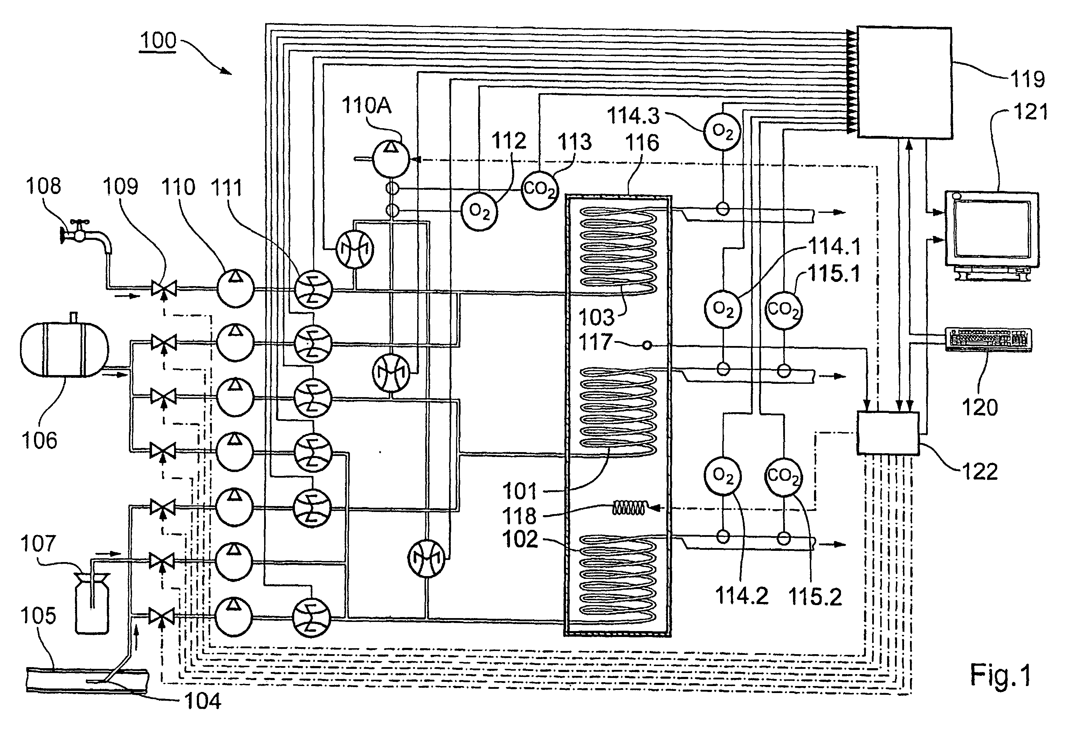

Fig. 1 zeigt eine Meßanordnung 100 zur Bestimmung der Nitrifikationsleistung des Belebtschlamms einer Kläranlage sowie des biochemischen Sauerstoffbedarfs und der Kohlenstoffbilanz eines Abwassers, die drei gleichartig aufgebaute und gleiche Abmessungen aufweisende Schlauchreaktoren 101, 102 und 103 aufweist. Der erste Schlauchreaktor 101 ist zulaufseitig mit einem Probenahmeröhrchen 104 verbunden, das in ein das zu untersuchende Abwasser führendes Rohr 105 ragt. Weiterhin ist sein Zulauf mit einer (hier symbolisch als Tank 106 dargestellten) Quelle für den Belebtschlamm verbunden. Der zweite Schlauchreaktor 102 ist zulaufseitig ebenfalls mit dem Probenahmeröhrchen 104 und dem Belebtschlamm-Tank 106 sowie zusätzlich mit einem Behälter 107 verbunden, der Allylthioharnstoff als Nitrifikationsinhibitor enthält. Der dritte Schlauchreaktor 103 schließlich ist ebenfalls mit dem Belebtschlamm-Tank 106, jedoch nicht mit dem Probenahmeröhrchen, sondern stattdessen mit einer Frischwasserzufuhr 108 verbunden. Alle drei Reaktoren haben eine Zuluftleitung, über die in den Zulauf der Reaktoren Druckluft eingespeist wird. Ablaufseitig münden alle Reaktoren - was in der Figur nicht gezeigt ist - in das Abwasserrohr 105 (stromabwärts des Probenahmröhrchens 104) oder direkt in die Kläranlage.

Auf der Einlaufseite ist in jedem Leitungszweig ein Absperrventil 109 und eine Förderpumpe 110 sowie - mit Ausnahme der mit dem Behälter 107 verbundenen Leitung - ein Durchflußmesser 111 vorgesehen. (Diese Komponenten sind der besseren Übersichtlichkeit der Figur halber nicht jeweils mit separaten Bezugsziffern bezeichnet, sondern nur einmal an der obersten Leitung zwischem der Frischwaserzufuhr 108 und dem dritten Reaktor 103.) Die Zuluftleitungen sind mit einer gemeinsamen Luftpumpe 110A verbunden, die aus der Atmosphäre ansaugt.

Weiterhin sind an der Zuluftleitung eine eingangsseitige Brennstoffzelle 112 und ein eingangsseitiger CO₂-Sensor 113 sowie auf der Auslaufseite aller Reaktoren (im Gasraum über dem Flüssigkeitsstrom) jeweils eine ausgangsseitige Brennstoffzelle 114. 1 bis 114.3 und beim ersten und zweiten Reaktor ein ausgangsseitiger CO₂-Sensor 115.1 und 115.2 angeordnet, die den Sauerstoff- und CO₂-Gehalt der Zuluft zu bzw. des Abgassstromes von den Reaktoren erfassen.

Die Reaktoren 101 bis 103 befinden sich ein einem wärmeisolierenden Gehäuse 116, in dem weiterhin ein Temperaturfühler 117 und eine - in der Figur als Heizwendel symbolisierte - T-Einstelleinrichtung 118 angeordnet sind.

Sämtliche Durchflußmesser, Brennstoffzellen und CO₂-Sensoren sind mit Dateneingängen einer Auswertungseinheit 119 verbunden, die eingangsseitig zudem mit einer Bedientastatur 120 als Befehlseingabeeinheit und ausgangsseitig mit einem Bildschirm 121 als Anzeigeeinheit sowie einem Mikrocontroller 122 verbunden ist. Der Mikrocontroller 119 ist bei der hier gezeigten Anordnung seinerseits über eine Datenleitung eingangsseitig mit dem T-Fühler 117 und ausgangsseitig über Steuerleitungen mit den Absperrventilen 109 sowie der T-Steuereinrichtung 118 verbunden. Die Komponenten 119 bis 122 können in der Praxis kostengünstig als Standard-PC-Konfiguration ausgeführt sein, wobei eine speziell auf das Verfahren zugeschnittene Auswertungs- und Steuerungs-Software implementiert ist.

Das Verfahren läuft folgendermaßen ab:

Nach Öffnung der Absperrventile 109 und Inbetriebnahme der Förderpumpen 110 und 110A werden die Einsatzstoffe in der durch die Leitungsverläufe vorgegebenen Zuordnung und in einer für jeden Reaktor und jeden Einsatzstoff (mit Ausnahme des Nitrifikationsinhibitors) durch die Durchflußmesser 111 spezifisch erfaßten Menge (pro Zeiteinheit) den Reaktoren 101 bis 103 zugeführt. Dabei wird an der Zuleitung kontinuierlich die Sauerstoff- und CO₂-Konzentration erfaßt. An den Ausgängen aller Reaktoren wird über die jeweiligen Brennstoffzellen kontinuierlich die Sauerstoffkonzentration über dem ausströmenden Reaktionsprodukt erfaßt, und an den Ausgängen des ersten und zweiten Reaktors zudem die CO₂-Konzentration.

Das ATH wird dem zweiten Reaktor 102 in einer Menge zudosiert, die eine sichere Hemmung der Nitrifikationsreaktionen gewährleistet. Im ersten Reaktor 101, dem kein ATH zugeführt wird, laufen diese Reaktionen jedoch - neben der Umsetzung der Kohlenwasserstoffe, die in beiden Reaktoren geschieht - in der gleichen Weie ab wie in der Kläranlage, die mit demselben Belebtschlamm arbeitet wie die Meßanordnung. Dem dritten Reaktor 103 wird nur reines Wasser ohne Nährstoffe zugeführt, so daß hier lediglich die endogene Atmung der Belebtschlamm-Mikroorganismen auftritt. Der in diesem Reaktor - als Differenz der durch die Fühler 112 und 114.3 registrierten O₂-Meßwerte - zu verzeichnende Abfall des Sauerstoffgehalts reflektiert den Sauerstoffverzehr infolge endogener Atmung, die Differenz der Meßwerte der Sauerstoffühler 112 und 114.2 am zweiten Reaktor reflektiert den Sauerstoffverzehr infolge endogener Atmung und Veratmung der Kohlenwasserstoffverbindungen im Abwasser, und die Differenz der Meßwerte der Fühler 112 und 114.1 am ersten Reaktor schließlich den Sauerstoffverzehr durch endogene Atmung, Umsetzung der Kohlenwasserstoffverbindungen und Nitrifikation. Die Differenz der Meßwerte der CO₂-Fühler 13 und 115.1 bzw. 115.2 schließlich ermöglicht eine Aussage zur C-Bilanz der Reaktion und damit zusätzliche Schlüsse über die organischen Inhaltsstoffe des Abwassers.

Die Verarbeitung der Meßwerte mit den Durchflußmengen-Werten der Einsatzstoffe in der Auswertungseinheit anhand eines geeigneten Algorithmus (unter Einschluß der genannten Differenzwertbildungen) liefert daher laufend aktuelle spezifische Sauerstoffbilanzen für die genannten Prozesse sowie eine C-Bilanz, aus denen zum einen auf den Metabolismus bzw. die aktuelle Nitrifikationsleistung des Belebtschlamms und zum anderen auf den aktuellen BSB und die organischen Inhaltstoffe des Abwassers geschlosssen werden kann. Stehen Modell- und/oder Vergangenheitsdaten zur Kalibrierung zur Verfügung, sind die genannten wichtigen Größen des Belebtschlamms bzw. des aufzubereitenden Abwassers auf dem Bildschirm direkt darstellbar. Anhand der Auswertungsergebnisse können zudem - über den Controller 122 - die Verfahrensbedingungen des Meßverfahrens modifiziert werden, um etwa verfälschende Limitierungen des Nitrifikationsumsatzes durch die Menge oder Zusammensetzung der Einsatzstoffe zu vermeiden oder die Empfindlichkeit für die eine oder andere Meßgröße zu maximieren. Erforderlichenfalls können aus (in der Figur nicht dargestellten) Vorratsbehältern dem Abwasser gesteuert zusätzlich Nährstoffe zugeführt werden. Die aktuellen Parameter des Meßverfahrens sind auf dem Bildschirm 121 darstellbar, und über die Tastatur kann der Bediener sowohl in den Verfahrensablauf als auch in die Auswertung und Darstellung der Auswertungsergebnisse eingreifen. Die Verfahrenparameter für die Umsetzungen in dem ersten und zweiten Reaktor werden - insbesondere hinsichtlich der Mengenverhältnisse der Einsatzstoffe und der Temperatur - zweckmäigerweise möglichst den Bedingungen in der Kläranlage angenähert.

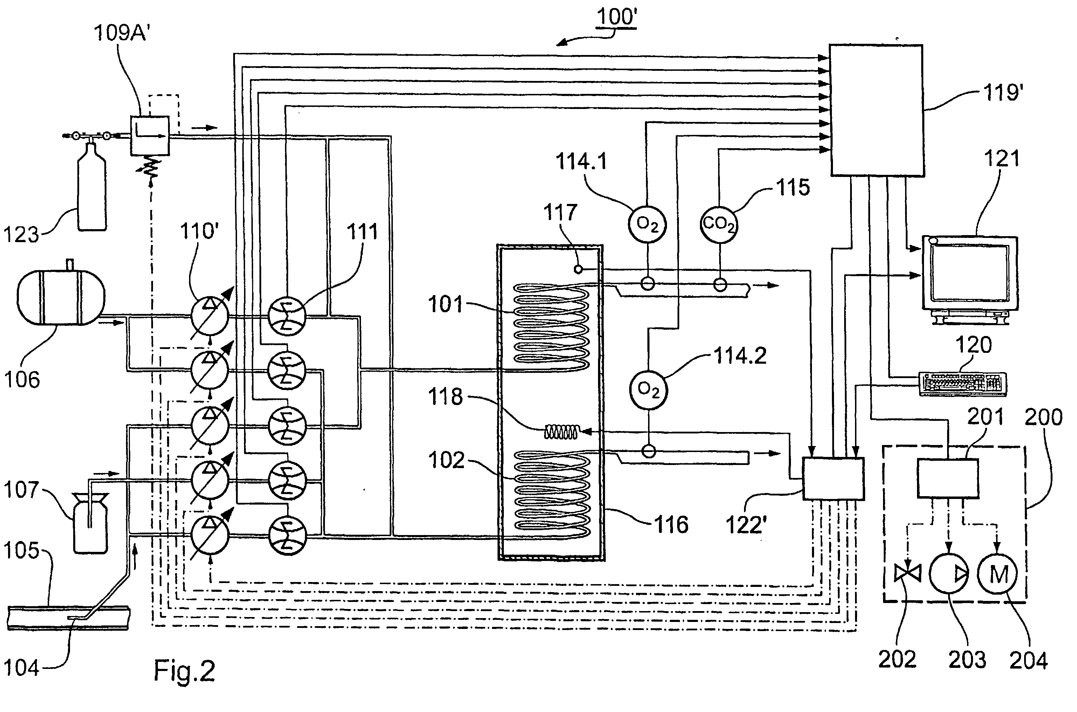

Fig. 2 zeigt eine gegenüber der Anordnung nach Fig. 1 modifizierte Meßanordnung 100'. Ein großer Teil der Komponenten entspricht jedoch den in Fig. 1 gezeigten; diese sind mit denselben Bezugsziffern wie dort bezeichnet und werden nachfolgend nicht nochmals erläutert. Ein wesentlicher Unterschied besteht im Fortfall des dritten Reaktors, ein weiterer in der Ersetzung der Luftzufuhr aus der Atmosphäre durch eine Zufuhr von Sauerstoff aus einer Druckflasche 123 über ein steuerbares Dosierventil 109A'. Absperrventile sind bei der vorliegenden Ausführung nicht vorgesehen, und die Förderpumpen sind als Dosierpumpen 110' ausgelegt. Da der Sauerstoffgehalt des zugeführten Gases hier genau bekannt ist (nahe 100% - entsprechend Spezifikation durch den Lieferanten) und CO₂ in diesem nicht enthalten ist, wird auf die einlaufseitigen O₂- und CO₂-Fühler verzichtet.

Für die Auswertung wird - was den Fortfall des dritten Reaktors ermöglicht - der Sauerstoffverbrauch durch endogene Atmung des Belebtschlamms (bakterielle Respiration) als bekannt vorausgesetzt, so daß der (zumindest hinsichtlich der eingesetzten Software natürlich ebenfalls modifizierten) Auswertungseinheit 119' neben den Durchflußmengenwerten nur die von den auslaufseitigen O₂-Fühlern und einem auslaufseitigen CO₂-Fühler 115 ermittelten Meßwerte zugeführt werden. Die Auswertung erfolgt gemäß dem oben erläuterten Prinzip und führt grundsätzlich zu den gleichen Aussagen - mit dem Unterschied, daß an die Stelle einiger Meßwerte vorgegebene Werte treten. Die Auswertungseinheit 119' ist hier zusätzlich mit einem Eingang einer Prozeßsteuereinheit 201 der Kläranlage 200 verbunden, so daß anhand der Auswertungsergebnisse unmittelbar in die Steuerung des Klärprozesses eingegriffen werden kann. Dies ist in der Figur dadurch symbolisiert, daß die Prozeßsteuereinheit ihrerseits mit einem Absperrventil 202, einer Förderpumpe 203 sowie einem Stellmotor 204 verbunden gezeigt ist. Durch Betätigung derartiger Stellmittel können beispielsweise in Abhängigkeit von der erfaßten aktuellen Nitrifikationsleistung des Belebtschlamms bzw. von deren zeitlicher Entwicklung ein Mittel zur Einstellung des pH-Wertes zugeführt oder Maßnahmen zur Eliminierung von die Nitrifikation beeinträchtigenden toxischen Stoffen ergriffen werden.

Die in Fig. 2 gezeigte Meßanordnung kann weiter vereinfacht werden, etwa indem auch auf die Durchflußmengenmesser verzichtet wird und der Auswertungseinheit stattdessen die Steuersignale des Controllers für die Dosierpumpen direkt zugeführt werden. Unter bestimmten Umständen kann auch eine aktive T-Regelung verzichtbar sein, wenn nur gewährleistet ist, daß die Reaktoren im wesentlichen die gleiche Prozeßtemperatur haben. Die Schlauchreaktoren können durch - an sich bekannte - Kaskaden aus (bevorzugt drei oder vier) Rührreaktoren ersetzt sein. Wenn lediglich Aussagen zur Nitrifikationsleistung und zum BSB, nicht aber eine Kohlenstoffbilanz, benötigt werden, ist eine Erfassung und Auswertung des CO₂-Gehaltes verzichtbar. Mit der Zwei-Reaktoren-Anordnung gemäß Fig. 2 kann im Prinzip auch gearbeitet werden, wenn der Sauerstoffverbrauch infolge endogener Atmung nicht bekannt ist. Erfolgt dann die Beschickung auf die in Fig. 2 dargestellte Weise, so ist in diesem Fall aber der BSB nicht separat ermittelbar. Es ist aber möglich, den Sauerstoffbedarf für die bakterielle Respiration in derselben Anlage durch eine getrennte Kalbriermessung (bevorzugt vorab) zu bestimmen und den so bestimmten Wert - zumindest als Näherungswert - für den nachfolgenden kontinuierlichen Meßprozeß zu nutzen.

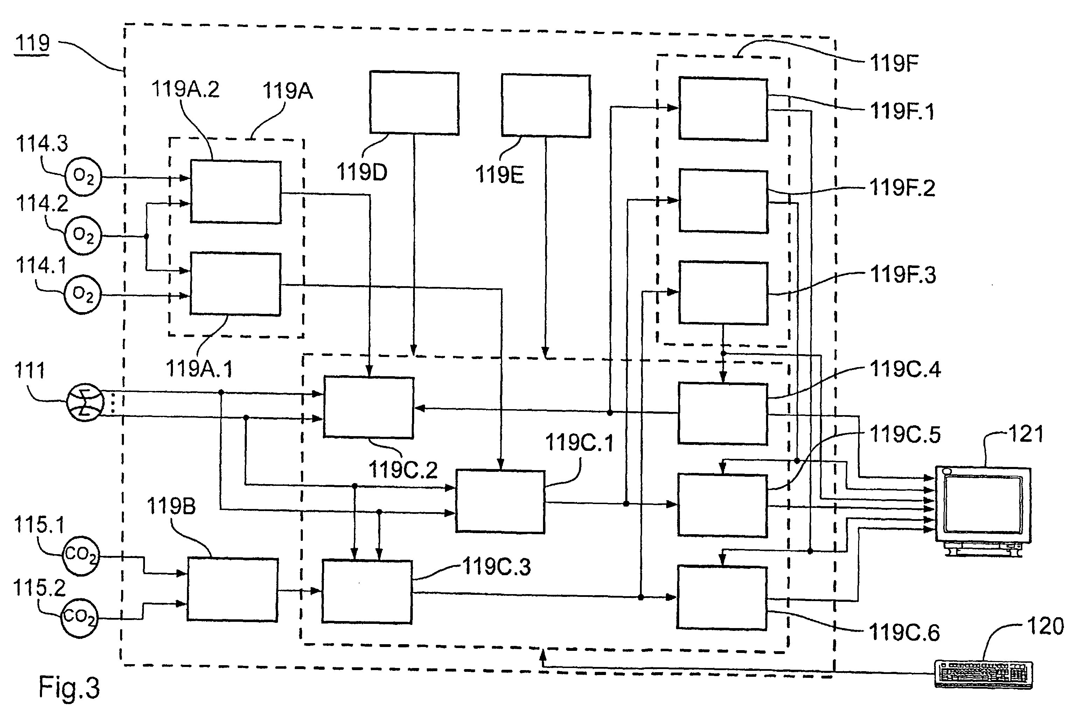

Fig. 3 zeigt in Gestalt eines Funktions-Blockschaltbildes wesentliche Funktionen bzw. Komponenten einer Auswertungseinheit am Beispiel der Auswertungseinheit 119 aus Fig. 1.

Diese umfaßt eine mit den O₂-Sensoren 114.1 bis 114.2 verbundene Subtraktionsstufe 119A, die zwei separate Funktionseinheiten umfaßt: eine erste Subtraktionseinheit 119A1, die mit den O₂-Fühlern 114.1 und 114.2 verbunden ist und die Differenz der Sauerstoffgehaltswerte am ersten und zweiten Reaktor bildet, und eine zweite Subtraktionseinheit 199A.2, die mit den O₂-Fühlern 114.2 und 114.3 verbunden ist und die Differenz der Sauerstoffgehaltswerte am zweiten und dritten Reaktor bildet. Sie umfaßt weiter eine mit den CO₂-Fühlern 115.1 und 115.2 verbundene Einheit 119B zur Plausibilitätsprüfung der CO₂-Meßwerte.

Die Ausgänge aller genannten Einheiten sowie der Durchflußmengenmesser 111 sind mit einer Verarbeitungseinheit 119C verbunden, der weiterhin - in üblicher Weise - die Eingabetastatur 120 sowie ein Programmspeicher 119D und ein Arbeitsspeicher 119E zugeordnet sind. Die Verarbeitungseinheit 119C umfaßt eine erste Berechnungsstufe 119C.1 zur Berechnung des (normierten) aktuellen Nitrifikations-Sauerstoffbedarfs, eine zweite Berechnungsstufe 119C.2 zur Berechnung des normierten aktuellen BSB und eine dritte Berechnungsstufe 119C.3 zur Berechnung der normierten aktuellen C-Bilanz. Die Ausgänge dieser Stufen sind mit jeweils einem FIFO-Speicher 119F.1, 119F:2 und 119F.3 einer Vergleichswert-Speichereinheit 119F verbunden, in denen jeweils eine fortlaufend aktualisierte Menge von Vergleichswerten für die genannten Größen gespeichert ist. Weiterhin sind die Berechnungsstufen mit jeweils einer Trendberechnungseinheit 119C.4, 119C.5 bzw. 119C.6 verbunden, in denen anhand der aktuellen Werte und der in den Speichern 119F.1 bis 119F.3 gespeicherten Vergangenheitswerte Trendanalysen ausgeführt werden, die eine Beobachtung der Abwasserzusammensetzung im zeitlichen Verlauf und Rückschlüsse auf zeitabhängige Vorgänge im der Kläranlage, insbesondere im Belebtschlamm, erlauben. Mindestens ein Teil der gespeicherten Werte sowie die Ergebnisse der Trendberechnungen werden zuammen mit den aktuellen Werten der relevanten Grö0en auf dem Bildschirm 121 dargestellt.

Die Erfindung beschränkt sich in ihrer Ausführung nicht auf die vorstehend angegebenen bevorzugten Ausführungsbeispiele. Vielmehr ist eine Vielzahl von Varianten denkbar, welche von der grundsätzlichen Lösung auch in anders gearteten Ausführungen Gebrauch macht.

## Patentansprüche

1. Verfahren zur Bestimmung der Nitrifikationsleistung von Belebtschlamm in einer wäßrigen Lösung, insbesondere mit Kohlenwasserstoffen belastetem Wasser, mit den Schritten:
- Zuführen einer jeweils vorbestimmten oder meßtechnisch erfaßten Menge des Belebtschlamms, der wäßrigen Lösung und von Luft oder Sauerstoff zu einem ersten und einem zweiten Reaktionsgefäß,
- gleichzeitiges Zuführen eines Nitrifkationsinhibitors nur zu dem zweiten Reaktionsgefäß,
- Ausführen einer Veratmungsreaktion in dem ersten und zweiten Reaktionsgefäß für eine vorbestimmte Zeitdauer oder kontinuierlich mit einer pro Zeiteinheit bestimmten Menge an Belebtschlamm, wäßriger Lösung und Luft oder Sauerstoff,
- Messung des Sauerstoffgehaltes über der wäßrigen Lösung im ersten und zweiten Reaktionsgefäß nach gleicher Reaktionszeit in beiden Reaktionsgefäßen oder kontinuierlich in einem aus dem jeweiligen Reaktionsgefäß entweichenden Abluftstrom,
- vergleichende Auswertung der am ersten und zweiten Reaktionsgefäß ermittelten Sauerstoffgehalts-Meßwerte unter Berücksichtigung der Mengen der Einsatzstoffe und unter Einschluß einer Differenzbildung aus den Sauerstoffgehalts-Meßwerten oder aus den Werten einer jeweils von diesen abgeleiteten Größe.

2. Verfahren nach Anspruch 1, **dadurch gekennzeichnet, daß**
- zu einem dritten Reaktionsgefäß eine vorbestimmte oder meßtechnisch erfaßte Menge des Belebtschlamms und von Luft oder Sauerstoff sowie von reinem Wasser zugeführt wird,
- in dem dritten Reaktionsgefäß über die gleiche Zeitspanne wie im ersten und zweiten Reaktionsgefäß oder kontinuierlich mit einer pro Zeiteinheit bestimmten Menge an Belebtschlamm, Wasser und Luft oder Sauerstoff ebenfalls eine Veratmungsreaktion ausgeführt wird,
- am dritten Reaktionsgefäß nach gleicher Reaktionszeit wie beim ersten und zweiten Reaktionsgefäß oder kontinuierlich der Sauerstoffgehalt über dem Wasser oder in einem Abluftstrom aus diesem Reaktionsgefäß gemessen wird und
- der Sauerstoffgehalts-Meßwert am dritten Reaktionsgefäß in die vergleichende Auswertung einbezogen wird derart, daß die Auswertung eine Differenzbildung aus den am zweiten und dritten Reaktionsgefäß ermittelten Meßwerten oder zwei aus diesem Meßwerten bestimmten Werten einer abgeleiteten Größe zur Bestimmung des biochemischen Sauerstoffbedarfs der wäßrigen Lösung aufweist.

3. Verfahren nach Anspruch 1, **dadurch gekennzeichnet, daß** aus dem am zweiten Reaktionsgefäß ermittelten Sauerstoffgehalts-Meßwert unter Differenzbildung zu einem vorbestimmten Wert für den Sauerstoffgehalt oder -umsatz infolge endogener Atmung im Belebtschlamm der biochemische Sauerstoffbedarf der wäßrigen Lösung ermittelt wird.

4. Verfahren nach einem der vorhergehenden Ansprüche, **dadurch gekennzeichnet, daß** mindestens in einem des ersten und zweiten Reaktionsgefäßes zusätzlich der Kohlendioxidgehalt über der wäßrigen Lösung oder kontinuierlich im Abluftstrom gemessen und hieraus eine Kohlenstoffbilanz gewonnen wird.

5. Verfahren nach einem der vorhergehenden Ansprüche, **dadurch gekennzeichnet, daß** am Eingang der Reaktionsgefäße der Sauerstoffgehalt der Luft gemessen und die Auswertung aufgrund der Differenz zwischen ein- und ausgangseitigem Sauerstoffgehalt vorgenommen wird.

6. Verfahren nach einem der vorhergehenden Ansprüche, **dadurch gekennzeichnet, daß** das Verfahren kontinuierlich durchgeführt wird, wobei die pro Zeiteinheit den Reaktionsgefäßen zugeführten Mengen der Einsatzstoffe über Durchflußsteuerung oder -messung bestimmt werden.

7. Verfahren nach einem der vorhergehenden Ansprüche, **dadurch gekennzeichnet, daß** das erste und zweite und wahlweise vorgesehene dritte Reaktionsgefäß verfahrenstechnisch identisch ausgebildet sind.

8. Verfahren nach einem der vorhergehenden Ansprüche, **dadurch gekennzeichnet, daß** als Nitrifikationsinhibitor Allylthioharnstoff eingesetzt wird.

9. Verfahren nach einem der vorhergehenden Ansprüche, **dadurch gekennzeichnet, daß** dem ersten und zweiten Reaktionsgefäß mit der wäßrigen Lösung ein Nährstoff, insbesondere Ammoniumsalz, Harnstoff, ein Pepton, Zucker und/oder eine organische Säure, zugeführt wird.

10. Verfahren nach einem der vorhergehenden Ansprüche, **dadurch gekennzeichnet, daß** es bei einer im wesentlichen konstanten und für die Reaktionsgefäße gleichen Temperatur im Bereich zwischen 20°C und 40°C, bevorzugt zwischen 30°C und 35°C, durchgeführt wird.

11. Vorrichtung zur Durchführung des Verfahrens nach einem der vorhergehenden Ansprüche, **gekennzeichnet durch**
- mindestens ein erstes und zweites Reaktionsgefäß (101, 102), denen jeweils Zufuhreinrichtungen (104, 106, 110A, 123) zur Zuführung von wäßriger Lösung, Belebtschlamm und Luft oder Sauerstoff zugeordnet sind, wobei nur einem der Reaktionsgefäße (102) zusätzlich eine Einrichtung (107) zur Zuführung eines Nitrifikationsinhibitors zugeordnet ist,
- Mitteln (110, 111; 109A', 110')zur Vorbestimmung oder Messung der zugeführten Mengen an wäßriger Lösung, Belebtschlamm und Luft oder Sauerstoff,
- mindestens einem ersten und zweiten, dem ersten Reaktionsgefäß zugeordneten Sauerstoffühler (114.1, 114.2) zur Messung des Sauerstoffgehaltes über der wäßrigen Lösung oder in einem Abluftstrom im bzw. aus dem ersten und zweiten Reaktionsgefäß und
- einer mit den Mitteln zur Vorbestimmung oder Messung und den Fühlern verbundenen Auswertungseinrichtung (119; 119') zur Ermittlung des normierten Nitrifikations-Sauerstoffumsatzes, die eine Subtraktionseinheit (119A.1) zur Differenzbildung aus den Ausgangssignalen des ersten und zweiten Sauerstoffühlers oder aus von diesen abgeleiteten Signalen aufweist.

12. Vorrichtung nach Anspruch 11, **dadurch gekennzeichnet, daß** ein drittes Reaktionsgefäß (103), dem Zufuhreinrichtungen (106, 108, 110A) zum Zuführen von reinem Wasser, Belebtschlamm und Luft oder Sauerstoff zugeordnet sind, und ein dem dritten Reaktionsgefäß zugeordneter dritter Sauerstoffühler (114.3) vorgesehen sind und die Auswertungseinrichtung (119) zusätzlich zur Ermittlung des biochemischen Sauerstoffbedarfs ausgebildet ist und eine Subtraktionseinheit (119A.2) zur Differenzbildung aus den Ausgangssignalen des zweiten und dritten Sauerstoffühlers oder aus von diesen abgeleiteten Signalen aufweist.

13. Vorrichtung nach Anspruch 11, **dadurch gekennzeichnet, daß** die Reaktionsgefäße kontinuierlich durchströmte Schlauchreaktoren (101 bis 103) oder jeweils eine Reihe hintereinandergeschalteter Rührreaktoren mit gleichem Aufbau und gleichen Abmessungen sind.

14. Vorrichtung nach einem der Ansprüche 11 bis 13, **dadurch gekennzeichnet, daß** mindestens einem des ersten und zweiten Reaktionsgefäßes ein Kohlendioxidfühler (115.1, 115.2) zugeordnet und die Auswertungseinrichtung mit diesem verbunden und zur Bestimmung der Kohlenstoffbilanz der wäßrigen Lösung ausgebildet ist.

15. Vorrichtung nach einem der Ansprüche 11 bis 14, **dadurch gekennzeichnet, daß** die Mittel zur Vorbestimmung oder Messung Durchflußvolumenregler oder -messer (112; 109A') sind.

16. Vorrichtung nach einem der Ansprüche 11 bis 15, **dadurch gekennzeichnet, daß** das erste und zweite Reaktionsgefäß über ein Probenahmeröhrchen (104) direkt mit einer Wasser- oder Abwasserleitung (105) verbunden sind.

17. Vorrichtung nach einem der Ansprüche 11 bis 16, **dadurch gekennzeichnet, daß** die Reaktionsgefäße, bevorzugt gemeinsam, in einem Thermostaten (116) untergebracht sind.

18. Vorrichtung nach einem der Ansprüche 11 bis 17, **dadurch gekennzeichnet, daß** eine Ablaufsteuereinheit (122; 122') vorgesehen ist, die insbesondere die Zufuhr der Einsatzstoffe und den Betrieb der Auswertungseinrichtung sowie wahlweise die Verfahrenstemperatur steuert.

19. Vorrichtung nach einem der Ansprüche 11 bis 18, **dadurch gekennzeichnet, daß** auch am Eingang der Reaktionsgefäße ein Sauerstoffühler (112) und/oder ein Kohlendioxidfühler (113) vorgesehen sind.

20. Vorrichtung nach einem der Ansprüche 11 bis 19, **dadurch gekennzeichnet, daß** als Sauerstoffühler Brennstoffzellen vorgesehen sind.

## Claims

1. A method for determining the nitrification effectiveness of activated sludge in an aqueous solution, in particular water laden with hydrocarbons, having the following steps:
- supplying a predetermined or measured quantity of activated sludge to the aqueous solution and delivering air or oxygen to a first and a second reaction vessel,
- simultaneous delivery of a nitrification inhibitor to only the second reaction vessel,
- performing a respiration reaction in the first and second reaction vessels for a predetermined period of time or continuously, with a predetermined quantity of activated sludge, aqueous solution, and air or oxygen per unit of time,
- measuring the oxygen content above the aqueous solution in the first and second reaction vessel after the same reaction time in both reaction vessels, or continuously in a waste air stream escaping from the respective reaction vessel,
- comparatively evaluating the measured oxygen content values ascertained at the first and second reaction vessels, taking into account the quantities of starting materials and forming a difference between the measured oxygen content values or the values of a variable derived from them.

2. The method of claim 1, **characterized in that**
- a predetermined or measured quantity of the activated sludge and of air or oxygen and of pure water is supplied to a third reaction vessel,
- in the third reaction vessel, over the same period of time as in the first and second reaction vessels or continuously, a respiration action is again performed with a predetermined quantity of activated sludge, water, and air or oxygen per unit of time,
- at the third reaction vessel, after the same reaction time as for the first and second reaction vessels or continuously, the oxygen content above the water or in a waste air stream from this reaction vessel is measured, and
- the measured oxygen content value at the third reaction vessel is included in the comparative evaluation in such a way that the evaluation forms a difference between the measured values ascertained in the second and third reaction vessels or between two values, determined from these measured values, of a derived variable in order to determine the biochemical oxygen demand of the aqueous solution.

3. The method of claim 1, **characterized in that** from the measured oxygen content value ascertained at the second reaction vessel, with a difference being formed from a predetermined value for the oxygen content or oxygen reaction as a consequence of endogenous respiration in the activated sludge, the biochemical oxygen demand of the aqueous solution is ascertained.

4. The method of one of the preceding claims, **characterized in that** in at least one of the first and second reaction vessels, in addition the carbon dioxide content is measured above the aqueous solution or continuously in the waste air stream, and a carbon balance is obtained therefrom.

5. The method of one of the preceding claims, **characterized in that** the oxygen content of the air is measured at the inlet to the reaction vessels, and the evaluation is made on the basis of the difference between the oxygen content on the inlet side and that on the outlet side.

6. The method of one of the preceding claims, **characterized in that** the method is performed continuously, and the quantities of starting materials supplied to the reaction vessels per unit of time are determined by way of flow control or flow measurement.

7. The method of one of the preceding claims, **characterized in that** the first and second and optionally provided third reaction vessel are embodied identically from the standpoint of process technology.

8. The method of one of the preceding claims, **characterized in that** allylthiourea is used as the nitrification inhibitor.

9. The method of one of the preceding claims, **characterized in that** a nutrient, in particular ammonium salt, urea, a peptone, sugar, and/or an organic acid, is added to the first and second reaction vessels along with the aqueous solution.

10. The method of one of the preceding claims, **characterized in that** it is performed at an essentially constant temperature, which is the same for all the reaction vessels, in the range between 20°C and 40°C, preferably between 30°C and 35°C.

11. An apparatus for performing the method of one of the preceding claims, **characterized by**
- at least one first and second reaction vessel (101, 102), to each of which delivery means (104, 106, 110A, 123) are assigned for delivering aqueous solution, activated sludge and air or oxygen, only one of the reaction vessels (102) additionally being assigned a device (107) for delivering a nitrification inhibitor,
- means (110, 111; 109A', 110') for predetermining or measuring the supplied quantities of aqueous solution, activated sludge and air or oxygen,
- at least one first and second oxygen sensor (114.1, 114.2), assigned to the first reaction vessel, for measuring the oxygen content above the aqueous solution in the first and second reaction vessels, or in a waste air stream from them, and
- an evaluation device (119; 119'), connected to the predetermining or measuring means and to the sensors, for ascertaining the standardized nitrification-oxygen reaction, which has a subtraction unit (119A.1) for forming the difference between the output Signals of the first and second oxygen sensors or between signals derived from them.

12. The apparatus of claim 11, **characterized in that** a third reaction vessel (103), to which delivery devices (106, 108, 110A) for delivering pure water, activated sludge and air or oxygen are assigned, and a third oxygen sensor (114.3) assigned to the third reaction vessel are provided, and the evaluation device (119) is additionally embodied for ascertaining the biochemical oxygen demand and has a subtraction unit (119A.2) for forming the difference between the output signals of the second and third oxygen sensors or between signals derived from them.

13. The apparatus of claim 11, **characterized in that** the reaction vessels are tubular reactors (101-103) with a continuous flow through them, or are each a series of agitator reactors, connected one after the other, of the same construction and the same dimensions.

14. The apparatus of one of claims 11 to 13, **characterized in that** a carbon dioxide sensor (115.1, 115.2) is assigned to at least one of the first and second reaction vessels, and the evaluation device communicates with it and is embodied to determine the carbon balance of the aqueous solution.

15. The apparatus of one of claims 11 to 14, **characterized in that** the predetermining or measuring means are flow volume regulators or flow volume meters (112; 109A').

16. The apparatus of one of claims 11 to 15, **characterized in that** the first and second reaction vessels communicate directly, via a sampling tubule (104) , with a water or wastewater line (105).

17. The apparatus of one of claims 11 to 16, **characterized in that** the reaction vessels are accommodated, preferably in common, in a thermostat (116).

18. The apparatus of one of claims 11 to 17, **characterized in that** a sequence control unit (112; 122') is provided, which in particular controls the delivery of starting materials and the operation of the evaluation device and selectively also controls the process temperature.

19. The apparatus of one of claims 11 to 18, **characterized in that** an oxygen sensor (112) and/or a carbon dioxide sensor (113) is also provided at the inlet to the reaction vessels.

20. The apparatus of one of claims 11 to 19, **characterized in that** fuel cells are provided as the oxygen sensor.

## Revendications

1. Procédé pour déterminer la puissance de nitrification de boue activée dans une solution aqueuse, en particulier d'eau chargée de carbures d'hydrogène, comportant les étapes :
apport d'une quantité chaque fois prédéterminée ou définie par une technique de mesure de la boue activée, de la solution aqueuse et d'air ou d'oxygène vers un premier et un deuxième récipient de réaction,
apport simultané d'un inhibiteur de nitrification seulement au deuxième récipient de réaction,
exécution d'une aérobiose dans le premier et le deuxième récipient de réaction pendant une durée déterminée ou de façon continue avec une quantité déterminée par unité de temps de boue activée, de solution aqueuse et d'air ou d'oxygène,
mesure de la teneur en oxygène au travers de la solution aqueuse du premier et du deuxième récipient de réaction après un temps de réaction égal dans les deux récipients de réaction ou en permanence dans un courant d'échappement d'air sortant du récipient de réaction respectif,
exploitation comparative des valeurs mesurées de teneur en oxygène dans le premier et le deuxième récipient de réaction en tenant compte des quantités des produits mis en oeuvre et en incluant la formation d'une différence des quantités mesurées de teneur en oxygène ou des valeurs d'une grandeur chaque fois dérivée de celles-ci.

2. Procédé selon la revendication 1, **caractérisé en ce que**
une quantité prédéterminée ou définie par une technique de mesure de boue activée, d'air ou d'oxygène ainsi que d'eau pure est apportée vers un troisième récipient de réaction,
dans le troisième récipient de réaction, pendant la même durée que pour le premier et le deuxième récipient de réaction ou de façon continue avec une quantité déterminée par unité de temps de boue activée, d'eau et d'air ou d'oxygène, une aérobiose est également exécutée,
dans le troisième récipient de réaction, après un même temps de réaction que pour le premier et le deuxième récipient de réaction ou de façon continue, la teneur en oxygène est mesurée au travers de l'eau ou dans un courant d'échappement de ce récipient de réaction et
la valeur mesurée de teneur en oxygène du troisième récipient de réaction est incluse dans l'exploitation comparative de manière telle que l'exploitation de la formation de différence des valeurs mesurées acquises du deuxième et du troisième récipient de réaction ou de deux valeurs définies parmi ces valeurs mesurées présente une grandeur dérivée pour déterminer le besoin biochimique en oxygène de la solution aqueuse.

3. Procédé selon la revendication 1, **caractérisé en ce que** le besoin biochimique en oxygène de la solution aqueuse est évalué à partir de la valeur mesurée de teneur en oxygène acquise pour le deuxième récipient de réaction par formation de différence avec une valeur prédéterminée de teneur ou de transformation en oxygène du fait d'une respiration endogène dans la boue activée.

4. Procédé selon l'une des revendications précédentes, **caractérisé en ce qu**'au moins dans l'un des premier et deuxième récipients de réaction la teneur en dioxyde de carbone est en outre mesurée au travers de la solution aqueuse ou en permanence dans un courant d'échappement et qu'un bilan de carbone en est déduit.

5. Procédé selon l'une des revendications précédentes, **caractérisé en ce qu'**à l'entrée des récipients de réaction la teneur en oxygène de l'air est mesurée et que l'exploitation en est entreprise sur la base de la différence entre les teneurs en oxygène en entrée et en sortie.

6. Procédé selon l'une des revendications précédentes, **caractérisé en ce que** le procédé est exécuté en permanence, les quantités des produits mis en oeuvre apportées par unité de temps aux récipients de réaction sont déterminées par des mesures ou des commandes d'écoulement.

7. Procédé selon l'une des revendications précédentes, **caractérisé en ce que** le premier et le deuxième récipient de réaction ainsi que le troisième qui est lui facultatif sont conformés de manière identique quant à la technique de procédé.

8. Procédé selon l'une des revendications précédentes, **caractérisé en ce que** de la thio-urée allylique est mise en oeuvre en tant qu'inhibiteur de nitrification.

9. Procédé selon l'une des revendications précédentes, **caractérisé en ce que** dans le premier et le deuxième récipient de réaction qui contiennent la solution aqueuse, un produit nutritif, en particulier du sel d'ammonium, de l'urée, une peptone, du sucre et/ou un acide organique sont apportés.

10. Procédé selon l'une des revendications précédentes, **caractérisé en ce qu'**il est appliqué à une température essentiellement constante et égale pour les récipients de réaction, dans la plage des 20 °C à 40 °C, de manière préférée entre 30 °C et 35 °C.

11. Appareil pour appliquer le procédé selon l'une des revendications précédentes, **caractérisé par**
au moins un premier et un deuxième récipient de réaction (101, 102), à chacun desquels sont affectés les dispositifs d'alimentation (104, 106, 110A, 123) pour apporter de la solution aqueuse, de la boue activée et de l'air ou de l'oxygène, dans lesquels un dispositif (107) supplémentaire d'apport d'inhibiteur de nitrification est affecté à un seul des récipients de réaction (102),
des moyens (110, 111 ; 109A', 110') de prédétermination ou de mesure des quantités apportées de solution aqueuse, de boue activée, d'air ou d'oxygène,
au moins un premier et un deuxième capteur d'oxygène (114.1, 114.2) affectés au premier récipient de réaction pour mesurer la teneur en oxygène au travers de la solution aqueuse ou dans un courant d'échappement d'air dans ou respectivement issu du premier et du deuxième récipient de réaction et
un dispositif d'exploitation (119 ; 119') lié avec les moyens de prédétermination ou de mesure et les capteurs pour acquérir la conversion standardisée en oxygène de nitrification et qui présente une unité de soustraction (119A.1) pour former la différence des signaux de sortie du premier et du deuxième capteur d'oxygène ou des signaux qui en sont dérivés.

12. Appareil selon la revendication 11, **caractérisé en ce qu'**un troisième récipient de réaction (103) auquel les dispositifs d'alimentation (106, 108, 110A) pour apporter de l'eau pure, de la boue activée et de l'air ou de l'oxygène sont affectés, et un troisième capteur d'oxygène (114.3) affecté au troisième récipient de réaction sont prévus et l'unité d'exploitation (119) est en outre formée pour l'acquisition du besoin biochimique en oxygène et présente une unité de soustraction (119A.2) pour former la différence des signaux de sortie du premier et du troisième capteur d'oxygène ou des signaux qui en sont dérivés.

13. Appareil selon la revendication 11, **caractérisé en ce que** les récipients de réaction sont des réacteurs à tuyau (101 à 103) traversés en permanence par le flux, ou chaque fois une série de réacteurs à agitateur raccordés les uns après les autres ayant la même structure et les mêmes dimensions.

14. Appareil selon l'une des revendications 11 à 13, **caractérisé en ce qu'**au moins à l'un des premier et deuxième récipients de réaction un capteur de dioxyde de carbone (115.1, 115.2) est affecté et que l'unité d'exploitation lui est reliée et est formée pour déterminer le bilan en carbone de la solution aqueuse.

15. Appareil selon l'une des revendications 11 à 14, **caractérisé en ce que** les moyens de prédétermination ou de mesure sont des régulateurs ou des compteurs volumétriques (112 ; 109A').

16. Appareil selon l'une des revendications 11 à 15, **caractérisé en ce que** le premier et le deuxième récipient de réaction sont directement reliés à une canalisation d'arrivée ou d'évacuation d'eau (105) au moyen de tubulures de prélèvement d'échantillons (104).

17. Appareil selon l'une des revendications 11 à 16, **caractérisé en ce que** les récipients de réaction sont installés de préférence ensemble dans un thermostat (116).

18. Appareil selon l'une des revendications 11 à 17, **caractérisé en ce qu'**une unité de contrôle d'écoulement (122 ; 122') est prévue, qui, en particulier, pilote l'apport des produits mis en oeuvre et le fonctionnement du dispositif d'exploitation ainsi que de manière sélective la température du procédé.

19. Appareil selon l'une des revendications 11 à 18, **caractérisé en ce qu'**également à l'entrée des récipients de réaction, un capteur d'oxygène (112) et/ou un capteur de dioxyde de carbone (113) sont prévus.

20. Appareil selon l'une des revendications 11 à 19, **caractérisé en ce que** des cellules à combustible sont prévues en tant que capteur d'oxygène.
